# EUROPÄISCHE PATENTANMELDUNG

(11) **EP 4 420 591 A1**
(43) Veröffentlichungstag der Anmeldung: **28.08.2024**
(21) Anmeldenummer: 24156445.9
(22) Anmeldetag: 07.02.2024
(51) Int. Cl.: A61B 1/018, A61B 1/317, A61B 18/22, A61B 18/24, A61M 1/00, A61B 1/00, A61B 1/015, A61B 1/06

(54) **ENDOSKOP UND VERFAHREN ZUM BETREIBEN**

(30) Priorität: 23.02.2023 DE 102023104405
(71) Anmelder: Laser Zentrum Hannover e.V., 30419 Hannover (DE)
(72) Erfinder: SPOIDA, Björn, 30559 Hannover (DE); RIPKEN, Tammo, 31515 Wunstorf (DE); JOHANNSMEIER, Sonja, 30163 Hannover (DE)
(74) Vertreter: Gramm, Lins & Partner Patent- und Rechtsanwälte PartGmbB

(57) **Zusammenfassung**

Die Erfindung betrifft ein Endoskop mit einem äußeren Hüllrohr (14), in dem ein Arbeitskanal mit einer vorderen Öffnung und einer hinteren Öffnung und einer Längsrichtung, die von der hinteren Öffnung zu der vorderen Öffnung verläuft, verläuft, wobei das Endoskop einen Arbeitstubus (2) mit einem vorderen Ende (4) und einem hinteren Ende aufweist, der in die hintere Öffnung des Arbeitskanals einschiebbar ist und wenigstens einen Lichtleitkanal (8) zum Leiten von Laserstrahlung, wenigstens einen Spülkanal (10) zum Leiten eines Spülgases und/oder einer Spülflüssigkeit und wenigstens einen Absaugkanal (6) aufweist.

## Beschreibung

Die Erfindung betrifft ein Endoskop mit einem äußeren Hüllrohr, in dem ein Arbeitskanal mit einer vorderen Öffnung und einer hinteren Öffnung und einer Längsrichtung, die sich von der hinteren Öffnung zu der vorderen Öffnung erstreckt, verläuft.

Derartige Endoskope sind aus dem Stand der Technik seit langem bekannt. Sie werden auch als medizinische Endoskope bezeichnet und beispielsweise für Operationen verwendet, bei denen die dem Patienten zugefügte Wunde möglichst klein sein soll. Man spricht hier von minimalinvasiver Medizin. Derartige Endoskope verfügen über einen Arbeitskanal, durch den das eigentliche medizinische Werkzeug, das für die jeweilige Operation benötigt wird, in den Körper des Patienten durch das Hüllrohr hindurch eingeführt wird. Oftmals findet sich in dem Hüllrohr zudem ein Beleuchtungskanal, durch den Licht einer oftmals externen Beleuchtungsquelle durch das Hüllrohr hindurch an die eigentliche Operationsstelle geleitet werden kann. In diesem Fall ist zudem in der Regel ein Beobachtungskanal vorhanden, durch den der Operateur durch das Hüllrohr hindurch die eigentliche Operationsstelle im Inneren des Körpers des Patienten beobachten kann, die durch Licht beleuchtet wird, dass durch den Beleuchtungskanal geleitet wurde.

Endoskope werden jedoch zudem bei anderen Operationen verwendet, bei denen enge und schmale Hohlräume durchstoßen werden müssen oder im Innern solcher Hohlräume gearbeitet werden muss. Dies ist beispielsweise der Fall, wenn eine Hüftprothese aus dem Knochen des Patienten entfernt werden soll. Eine Hüftprothese ist eine Endoprothese, die im Körper des Patienten angeordnet wird und das natürliche Hüftgelenk des Patienten ersetzt. Man bezeichnet sie daher auch oft als Hüfttotalendoprothese. Eine solche Prothese umfasst einen Prothesenschaft, der im Oberschenkelknochen des Patienten verankert wird, einen runden Prothesenkopf, der an dem proximalen, also dem Oberkörper zugewandten, Ende des Prothesenschaftes positioniert ist, ein daran angeordnetes Inlay, das als Gleitkomponente wirkt und ein Implantat der Hüftgelenkspfanne. Der Oberschenkelknochen ist ein Röhrenknochen, der folglich einen inneren Hohlraum aufweist, in den der Prothesenschaft der Hüftprothese eingeführt wird. Vorher wird der Hohlraum ausgefräst, um ausreichend Platz für den Prothesenschaft zu schaffen. Um eine möglichst gute Haftung zwischen dem eingesetzten Prothesenschaft und dem Knochen zu erreichen, wird in vielen Operationen ein sogenannter Knochenzement zwischen dem Knochen und dem Prothesenschaft eingesetzt, der wie ein Klebstoff wirkt.

Es gibt eine Vielzahl von medizinischen Indikationen, beispielsweise Lockerungen der Prothese, die dazu führen, dass eine Hüftprothese oder eine andere Totalendoprothese ausgetauscht werden muss. In diesen Fall muss der Prothesenschaft aus dem Oberschenkelknochen entfernt und durch einen anderen Prothesenschaft ersetzt werden. Problematisch ist dabei der im Knochen verbleibende Knochenzement, der ebenfalls entfernt werden muss, bevor ein neuer Prothesenschaft eingesetzt werden kann. Dies geschieht beispielsweise mittels eines Meißels oder eines mit einer Klinge versehenen Werkzeugs. Problematisch hierbei und bei anderen Methoden ist, dass es beim Entfernen des jeweiligen Teils des enthaltenen Knochenzementes nicht möglich ist, die jeweils bearbeitete Stelle und Position zu beobachten. Daher wird oftmals nicht nur der enthaltene Knochenzement, sondern beispielsweise auch ein Teil des Knochens entfernt, was nachteilig für die Stabilität des Knochens ist und auch eine später eingesetzte weitere Hüftprothese negativ beeinflussen kann. Dies geschieht oftmals dann, wenn größere Zementbrocken abgeschlagen oder abgestellt werden. Bei derartigen mechanischen Verfahren ist zudem die mechanische Belastung durch die aufgebrachten Kräfte sehr hoch, was ein zusätzliches Risiko für die Stabilität der verbleibenden Knochen darstellt.

Aus dem Stand der Technik sind eine ganze Reihe von Vorrichtungen bekannt, mit denen auf andere Weise der Knochenzement entfernt werden soll. Insbesondere ist versucht worden, dies durch eine Laserablation zu erreichen, bei der der Zement mit Laserstrahlung bestrahlt und so entfernt wird. Derartige Verfahren haben jedoch den Nachteil, dass die thermische Belastung von umgebendem Gewebe oder Knochenmaterial sehr hoch ist und es dadurch zu dauerhaften Schädigungen kommen kann. Eine dafür vorgesehene Vorrichtung ist beispielsweise der WO 2011/109414 A2 zu entnehmen. Aus der DE 3 212 180 ist beispielsweise ein endoskopischer Laserkoagulator bekannt, bei dem mittels einer Laserstrahl-Leitungsfaser Laserstrahlung an das vordere Ende des Endoskopes geleitet wird, wobei das Laserlicht nicht entlang der Längsrichtung des Endoskopes, sondern unter einem Winkel von 6° zu dieser Längsrichtung austritt. Die US 6, 620, 154 B1 zeigt eine ähnliche Vorrichtung, bei der am vorderen Ende des Endoskopes ein optisches Element angeordnet ist, mit dem das Laserlicht, das durch das Endoskop geleitet wird, unter einem Winkel von 90° zur Längsrichtung des Endoskopes austritt. Die Vorrichtung, die in dieser Druckschrift beschrieben wird, ist für die Operation von Augen vorgesehen.

Nachteilig ist, dass das Entfernen des im Knochen verbliebenen Knochenzementes auf mechanischen Weg durch Hammer und Meißel oftmals ungenau und schlecht kontrollierbar ist und daher vermehrt zu Beschädigungen des Knochens führt, die die Implantation einer Folge-Prothese erschweren. Das Entfernen mittels Laserstrahlung ist hingegen aufwendig und zeitintensiv. Eine lang andauernde Operation ist sowohl für den Operateur von Nachteil, da sie anstrengend ist und er ermüdet, als auch für den Patienten ungünstig, da die dafür notwendige lange Narkose die Risiken für den Patienten erhöht. Schließlich ist eine lange Operation auch aus wirtschaftlicher Sicht nachteilig, da die Kosten der Operation mit steigender Dauer ansteigen. Beim Verdampfen des Knochenzementes entstehen zudem giftige oder zumindest gesundheitsschädliche Gase, die aus einem Operationssaal sorgfältig entfernt werden müssen. Der Erfindung liegt daher die Aufgabe zugrunde, die Nachteile aus dem Stand der Technik zu beheben oder zumindest zu mildern.

Die Erfindung löst die gestellte Aufgabe durch ein Endoskop gemäß dem Oberbegriff des Anspruchs 1, das sich dadurch auszeichnet, dass es einen Arbeitstubus mit einem vorderen Ende und einem hinteren Ende aufweist, der in die hintere Öffnung des Arbeitskanals einschiebbar ist und wenigstens einen Lichtleitkanal zum Leiten von Laserstrahlung, wenigstens einen Spülkanal zum Leiten eines Spülgases und/oder einer Spülflüssigkeit und wenigstens einen Absaugkanal aufweist.

Die vordere Öffnung des Arbeitskanals befindet sich im Gebrauch des Endoskopes im Innern des Patienten, beispielsweise im Inneren eines Oberschenkelknochens. Die hintere Öffnung, durch die der Arbeitstubus in das Endoskop eingeschoben werden kann, ist auch während der Operation für den Operateur zugänglich. Auf diese Weise ist es möglich, den Arbeitstubus während der Operation aus dem Hüllrohr des Endoskopes zu entfernen oder ihn in das Hüllrohr einzuführen. So kann beispielsweise ein Arbeitstubus durch einen anderen Arbeitstubus ersetzt werden. Der Arbeitstubus enthält einen Lichtleitkanal, durch den Laserstrahlung, die vorzugsweise von einem außerhalb des Endoskopes befindlichen Lasers erzeugt wird, durch den Arbeitstubus hindurch an die gewünschte Stelle, beispielsweise im Innern des Oberschenkelknochens, geleitet wird. Diese Laserstrahlung kann verwendet werden, um beispielsweise Knochenzement oder eine andere Substanz mit dem Laser zu bearbeiten, beispielsweise zu entfernen oder zu verdampfen. Die dabei möglicherweise entstehenden Produkte, beispielsweise Gase, werden ebenfalls durch den Arbeitstubus ausgespült. Dazu wird ein Spülgas oder eine Spülflüssigkeit durch den wenigstens einen Spülkanal geleitet. Das Spülgas und/oder die Spülflüssigkeit treten durch das vordere Ende des Arbeitstubus und damit auch durch die vordere Öffnung des Arbeitskanals aus und spülen den sich davor befindenden Arbeitsraum. Das Spülgas und/oder die Spülflüssigkeit verlassen mit dem Material, welches durch das Spülen entfernt werden soll, den Arbeitsraum durch den wenigstens einen Absaugkanal.

In einer bevorzugten Ausgestaltung verfügt das hintere Ende des Arbeitstubus über eine Verbindungeinrichtung, mit der das hintere Ende des Lichtleitkanals mit einem Lichtleiter, beispielsweise einer Glasfaser, verbindbar ist, durch das Laserstrahlung von einem externen Laser zu dem Arbeitstubus hin geleitet werden kann. Diese Verbindungeinrichtung ist bevorzugt direkt am Lichtleitkanal, besonders bevorzugt an dessen hinteren Ende, befestigt. Vorzugsweise verfügt der Arbeitstubus über wenigstens eine Verbindungeinrichtung, mit der das hintere Ende des Spülkanals mit einer Gasleitung oder einer Flüssigkeitsleitung verbindbar ist. Durch diese Gasleitung oder Flüssigkeitsleitung wird ein Spülgas oder eine Spülflüssigkeit von einer externen Gasquelle oder Flüssigkeitsquelle zu dem Spülkanal geleitet. Diese Verbindungeinrichtung ist bevorzugt direkt am Spülkanal, besonders bevorzugt an dessen hinteren Ende, befestigt. Vorzugsweise verfügt der Arbeitstubus über wenigstens eine Verbindungeinrichtung, mit der das hintere Ende des Absaugkanals mit einer Absaugleitung verbindbar ist. Durch diese Absaugleitung lässt sich der Absaugkanal mit einer Sogquelle, beispielsweise einer Pumpe, verbinden, durch die die Absaugleitung mit einem Unterdruck beaufschlagbar ist, sodass Material, das sich vor dem vorderen Ende des Absaugkanals, also im Arbeitsraum, befindet, durch den Absaugkanal abgesaugt wird. Vorzugsweise befindet sich diese Verbindungeinrichtung direkt am Absaugkanal, besonders bevorzugt an dessen hinteren Ende.

Bevorzugt ist der Lichtleitkanal als Lichtleitfaser, insbesondere Glasfaser, und/oder als Hohlleiter und/oder als Freistrahl ausgebildet. Insbesondere für den Fall, dass eine Lichtleitfaser verwendet wird, ist es von Vorteil, wenn diese mit einer stabilisierenden Hülle, beispielsweise einem Metallrohr, umgeben ist.

In einigen Ausführungsformen der Erfindung ist der Lichtleitkanal zum vorderen Ende des Arbeitstubus hin durch ein optisches Bauteil, insbesondere eine Linse oder ein Prisma, verschlossen. Der Lichtleitkanal verfügt über ein vorderes Ende, das im Bereich des vorderen Endes des Arbeitstubus angeordnet ist. Das optische Bauteil kann auch als Lichtaustrittsfläche einer Lichtleitfaser oder als Durchtrittsfläche einer Lichtleitfaser, beispielsweise einer Glasfaser, ausgebildet sein. Durch das optische Bauteil wird der Lichtleitkanal zum vorderen Ende hin verschlossen, sodass insbesondere während einer Verwendung des Endoskopes, beispielsweise bei einer Operation, keine Flüssigkeiten und/oder Gase in den Lichtleitkanal eindringen können. Dies ist insbesondere dann wichtig und von Vorteil, wenn der Lichtleitfaser als Hohlleiter oder als Freistrahl ausgebildet ist. Ein möglichst hermetischer Verschluss des Lichtleitkanals erleichtert es, das Endoskop, zumindest jedoch den Arbeitstubus, nach dem Gebrauch zu reinigen und gegebenenfalls zu sterilisieren und so einer weiteren Verwendung zuführen zu können. Das optische Bauteil ist bevorzugt ein Kollimator oder ein fokussierendes Bauteil.

In einer bevorzugten Ausgestaltung ist das optische Bauteil so ausgebildet, dass Licht, das durch den Lichtleitkanal geleitet wird und durch das optische Bauteil hindurchtritt, entlang einer Austrittsrichtung aus dem optischen Bauteil austritt, die einen Winkel von wenigstens 10°, bevorzugt wenigstens 30° mit der Längsrichtung des Arbeitskanals einschließt. Vorzugsweise weist das Endoskop mehrere Arbeitstuben auf, die jeweils über ein optisches Bauteil am vorderen Ende des Lichtleitkanals verfügen, die unterschiedlichen Austrittsrichtungen erzeugen. Auf diese Weise lässt sich bei einer Operation zunächst ein erster Arbeitstubus verwenden, dessen optisches Bauteil am vorderen Ende des Lichtleitkanals eine erste Austrittsrichtung erzeugt. Zu einem späteren Zeitpunkt während der Operation kann dieser erste Arbeitstubus aus der hinteren Öffnung des Arbeitskanals des Endoskopes herausgezogen und durch einen zweiten Arbeitstuben ersetzt werden. Dieser zweite Arbeitstubus wird dann in die hintere Öffnung des Arbeitskanals eingeschoben. Vorzugsweise verfügt der zweite Arbeitstubus über ein optisches Bauteil am vorderen Ende des Lichtleitkanals, dass eine zweite Austrittsrichtung für das Laserlicht erzeugt, die von der ersten Austrittsrichtung verschieden ist.

In einer bevorzugten Ausgestaltung erstreckt sich die Austrittsrichtung in einem Winkel von weniger als 10°, vorzugsweise parallel zur Längsrichtung des Arbeitskanals. Dies ist insbesondere für den Fall von Vorteil, wenn eine Stelle bearbeitet werden soll, die in Längsrichtung vor dem vorderen Ende des Arbeitstubus liegt. Eine Ausgestaltung der Erfindung beinhaltet einen ersten Arbeitstubus, bei dem die Austrittsrichtung in einem Winkel von weniger als 10°, vorzugsweise 0° zur Längsrichtung des Arbeitskanals verläuft, und einen zweiten Arbeitstubus, bei dem die Austrittsrichtung in einem Winkel von etwa 30° zu dieser Längsrichtung des Arbeitskanals verläuft.

Das optische Bauteil kann auf unterschiedliche Weise ausgebildet sein. Vorzugsweise beinhaltet es einen Spiegel und/oder ein Prisma und/oder ein Gitter, die so angeordnet sind, dass Laserlicht, das durch den Lichtleitkanal geleitet wird, von diesem Bauteil abgelenkt wird. In einer bevorzugten Ausgestaltung ist das optische Bauteil beweglich ausgebildet, was beispielsweise durch einen verkippbaren und/oder drehbaren Spiegel erreicht werden kann. In dieser bevorzugten Ausgestaltung ist die Austrittsrichtung, entlang derer das Laserlicht, das durch den Lichtleitkanal geleitet wird, durch das optische Bauteil austritt, veränderbar und einstellbar.

In einer bevorzugten Ausgestaltung verläuft der Lichtleitkanal in einem Abschnitt des Arbeitstubus nicht parallel zum Arbeitstubus, wobei der Abschnitt vorzugsweise unmittelbar vor dem vorderen Ende des Arbeitstubus liegt. Dies ist insbesondere dann von Vorteil, wenn der Lichtleitkanal als Lichtleitfaser ausgebildet ist. Die Austrittsrichtung, in der Licht aus der Lichtleitfaser austritt, wird wesentlich bestimmt von der Richtung, entlang derer der Lichtleitkanal in dem letzten Abschnitt, durch den das Licht geleitet wird, verläuft. Der Abschnitt ist vorzugsweise einige Zentimeter, beispielsweise 2 cm, 3 cm oder 5 cm lang. In einer bevorzugten Ausgestaltung verläuft der Lichtleitkanal entlang des Abschnittes nicht gerade, sondern gekrümmt. Erweist in diesem Abschnitt vorzugsweise einen konstanten Krümmungsradius auf. Je länger der Abschnitt ausgebildet ist, desto größer kann der Krümmungsradius gewählt werden, wobei ein größerer Krümmungsradius vorteilhaft ist, da die Lichtleitfaser mechanisch weniger belastet wird und die Streuverluste durch die Mantelfläche der Lichtleitfaser austretende Laserstrahlung reduziert werden.

Vorteilhafterweise verändert sich in dem Abschnitt eine Form der Querschnittsfläche des Spülkanals und/oder des Absaugkanals, wobei vorzugsweise der Flächeninhalt der Querschnittsfläche konstant ist. Vorzugsweise verlaufen der Spülkanal und/oder der Absaugkanal parallel zum Arbeitstubus. Besonders bevorzugt verlaufen der Spülkanal und/oder der Absaugkanal über die gesamte Länge des Arbeitstubus parallel zum Arbeitstubus. Ist jedoch der Lichtleitkanal in dem bereits genannten Abschnitt nicht parallel zum Arbeitstubus, kann es notwendig oder zumindest vorteilhaft sein, wenn sich die Form der Querschnittsfläche des Spülkanals und/oder des Absaugkanals ändert, umso Platz für den jeweiligen Lichtleitkanal zu machen, der an unterschiedlichen Stellen in der Querschnittsfläche des Arbeitstubus verläuft.

In einer bevorzugten Ausgestaltung ist in dem äußeren Hüllrohr, vorzugsweise in dem Arbeitstubus, eine Beobachtungseinrichtung vorhanden, die eingerichtet ist, Licht zur detektieren, dass aus einem vorbestimmten Raumwinkelbereich relativ zur Längsrichtung des Arbeitskanals in die Beobachtungseinrichtung fällt. Vorzugsweise beinhaltet die Beobachtungseinrichtung einen Beobachtungskanal, der vorzugsweise als Lichtleitfaser, beispielsweise als Glasfaser, ausgebildet ist. Das Licht, das in dem Arbeitsraum vor der vorderen Öffnung des Arbeitskanals vorhanden ist und in dem jeweiligen Raumwinkelbereich in das vordere Ende der Lichtleitfaser eintritt, wird von dieser durch den Beobachtungskanal hindurchgeleitet. Es gelangt so beispielsweise auf einen optischen Sensor, beispielsweise einen CCD-Chip oder eine Kamera oder wird durch eine optische Einrichtung, beispielsweise ein Mikroskop, geleitet, sodass der Operateur, der das Endoskop verwendet, in den Arbeitsraum sehen kann.

Vorzugsweise ist der optische Sensor am vorderen Ende des äußeren Hüllrohrs, vorzugsweise am vorderen Ende des Arbeitstubus, angeordnet, sodass Licht direkt aus dem Arbeitsraum auf den optischen Sensor gelangen kann, ohne durch eine Lichtleitfaser geleitet zu werden. Die von dem optischen Sensor erzeugten Signale, die beispielsweise elektronische Signale sein können, werden durch eine Datenleitung an eine elektronische Datenverarbeitungseinrichtung übermittelt. Diese Datenleitung kann als Beobachtungskanal bezeichnet werden. Die Kommunikation zwischen dem optischen Sensor und der elektronischen Datenverarbeitungseinrichtung kann kabelgebundene erfolgen oder kabellos, beispielsweise über Funk, WiFi oder Bluetooth erfolgen.

Besonders bevorzugt liegt die Austrittsrichtung, entlang derer Licht aus dem Lichtleitkanal austritt, in dem vorbestimmten Raumwinkelbereich, wobei der vorbestimmten Raumwinkelbereich vorzugsweise symmetrisch um die Austrittsrichtung ist. Wenn die Austrittsrichtung in dem vorbestimmten Raumwinkelbereich liegt, ist sichergestellt, dass die Arbeitsstelle, an der das austretende Laserlicht auf einen Gegenstand oder ein Gewebe trifft, durch die Beobachtungseinrichtung beobachtbar ist. Auf diese Weise kann während der Verwendung des Endoskopes die Arbeitsstelle beobachtet werden.

Vorzugsweise weist die Beobachtungseinrichtung einen Lichtdetektor, beispielsweise einen CCD Chip auf. Dieser ist besonders bevorzugt so angeordnet, dass durch die vordere Öffnung eintretendes Licht unmittelbar oder durch eine optische Einrichtung fokussiert auf den Lichtdetektor auftrifft. Die so entstehenden elektrischen Signale werden durch eine vorhandene elektrische Leitung oder eine Signalleitung, die ebenfalls Teil der Beobachtungseinrichtung ist, durch das äußere Hüllrohr, vorzugsweise durch den Arbeitstubus, hindurch geleitet. Vorzugsweise verfügt das äußere Hüllrohr, besonders bevorzugt der Arbeitstubus über eine Verbindungeinrichtung, mit der die Verbindung mit einer externen Datenleitung hergestellt werden kann, über die die Signale und Arbeitsdaten, die durch den Lichtdetektor erzeugt wurden, einer Verarbeitungseinrichtung, vorzugsweise einer elektronischen Datenverarbeitungseinrichtung, zugeführt werden.

Es hat sich als vorteilhaft herausgestellt, wenn sich in dem Endoskop eine Beleuchtungseinrichtung befindet, die eingerichtet ist, einen Arbeitsraum, der sich vor der vorderen Öffnung des Arbeitskanals befindet, mit elektromagnetischer Strahlung zu beleuchten. Die Beleuchtungseinrichtung ist beispielsweise in Form einer Lichtleitfaser, beispielsweise einer Glasfaser, ausgebildet. Sie erstreckt sich durch den Arbeitskanal oder außerhalb des Arbeitskanals durch das Hüllrohr. Am der hinteren Öffnung des Arbeitskanals zugewandten Ende der Lichtleitfaser ist vorzugsweise eine Verbindungeinrichtung angeordnet, durch die Licht in die Lichtleitfaser eingeleitet werden kann. Unter Licht wird dabei jegliche elektromagnetische Strahlung verstanden, die zur Beleuchtung verwendet werden kann. Alternativ oder zusätzlich dazu verfügt die Beleuchtungseinrichtung über eine Lichtquelle, beispielsweise eine LED, die in dem äußeren Hüllrohr, beispielsweise im Arbeitskanal oder außerhalb des Arbeitskanals angeordnet ist und mit einer durch den Arbeitskanal oder außerhalb des Arbeitskanals aber innerhalb des Hüllrohrs laufenden elektrischen Leitung mit einer Stromversorgung verbunden ist. Auf diese Weise kann das zum Beleuchten benötigte Licht im Bereich der vorderen Öffnung des Arbeitskanals erzeugt werden und beleuchtet so vorzugsweise den gesamten Arbeitsraum.

Vorzugsweise sind der Arbeitskanal und/oder der Spülkanal und/oder der Absaugkanal in Form eines Rohres, vorzugsweise eines metallischen Rohres, ausgebildet. Das Rohr weist bevorzugt zumindest abschnittsweise, besonders bevorzugt jedoch über seine gesamte Länge, einen kreisförmigen Querschnitt auf. Der Querschnitt kann jedoch auch oval, ellipsoide oder frei geformt sein. Vorzugsweise verändert sich ein Durchmesser, ein Krümmungsradius und/oder eine geometrische Form des Querschnittes in Abhängigkeit der Position entlang der Längsrichtung des Arbeitskanals. Vorzugsweise bleibt der Flächeninhalt der Querschnittsfläche über die gesamte Länge des jeweiligen Kanals konstant.

In einer bevorzugten Ausgestaltung weist der Arbeitstubus mehrere, vorzugsweise wenigstens 2, wenigstens 3 oder wenigstens 4, Spülkanäle auf. Besonders bevorzugt sind diese mehreren Spülkanäle jeweils mit einer Quelle eines Spülgases oder einer Spülflüssigkeit verbunden. Vorteilhafterweise sind die einzelnen Spülkanäle mit jeweils einem Ventil versehen, durch das der Durchfluss des Spülgases und/oder der Spülflüssigkeit erlaubt und unterbunden werden kann. Besonders bevorzugt sind die Ventile unabhängig voneinander steuerbar, sodass die einzelnen Spülkanäle unabhängig voneinander geöffnet und geschlossen werden können. Damit lässt sich erreichen, dass Strömungsrichtung und/oder Strömungsgeschwindigkeit des Spülmediums innerhalb des Arbeitsraumes vor der vorderen Öffnung des Arbeitskanals verändert werden können. Stellt sich beispielsweise heraus, dass das verwendete Spülmediums ein beispielsweise aufgrund der Laserstrahlung entstehendes Gas so bewegt, dass nicht mehr ausreichend Licht in die Beobachtungseinrichtung eindringt, so kann die Anzahl oder die Auswahl der Spülkanäle, durch die das Spülmedium, also das Spülgas und/oder die Spülflüssigkeit, in den Arbeitsraum geleitet wird, verändert werden, indem einzelne Spülkanäle geschlossen und/oder geöffnet werden. Dadurch verändern sich die Strömungsgegebenheiten im Innern des Arbeitsraumes.

Vorteilhafterweise ist das vordere Ende des Arbeitstubus von einem Abschlussstück verschlossen, durch das alle Kanäle hindurchgeführt sind. Damit wird beispielsweise verhindert, dass Flüssigkeiten oder Gase durch die vordere Öffnung in den Arbeitskanal eindringen. Eine Reinigung und Desinfizierung des Endoskopes wird dadurch vereinfacht, sodass es schneller und mit weniger Aufwand wiederverwendet werden kann. Das Abschlussstück ist zudem eingerichtet, die jeweiligen Enden der Kanäle zueinander auszurichten und ihre Position relativ zueinander zu fixieren. Besonders bevorzugt ragt das Abschlussstück in Längsrichtung des Arbeitskanals über das optische Bauteil am vorderen Ende des Lichtleitkanals hinaus. Dadurch wird das oft empfindliche optische Bauteil vor mechanischen Beschädigungen geschützt.

Vorzugsweise weist der Arbeitstubus eine Arretierungseinrichtung auf, mit der er vorzugsweise in mehreren vorbestimmten Orientierungen, vorzugsweise einer einzigen Orientierung in dem Arbeitskanal arretierbar ist. Durch die Arretierbarkeit wird erreicht, dass das Endoskop als Ganzes, insbesondere mit dem sich darin befindenden Arbeitstubus bewegt werden kann. Das Endoskop verfügt vorzugsweise über einen Handgriff, an dem der Operateur das Endoskop anfassen und handhaben kann. Wird der Handgriff bewegt, der vorzugsweise starr mit dem äußeren Hüllrohr verbunden ist, wird auch dieses Hüllrohr bewegt. Dies hat zur Folge, dass auch der Arbeitstubus und die sich darin erstreckenden Kanäle und Einrichtungen bewegt werden, sofern der Arbeitstubus mit dem Arbeitskanal arretiert ist. Ist der Arbeitstubus nur in einer einzigen Orientierung relativ zum Arbeitskanal mit diesem arretierbar, lassen sich Fehleinstellungen und Fehl-Orientierungen sicher vermeiden.

Die Erfindung löst die gestellte Aufgabe zudem durch einen Arbeitstubus für ein hier beschriebenes Endoskop.

Die Erfindung löst die gestellte Aufgabe zudem durch ein Verfahren zum Betreiben eines hier beschriebenen Endoskopes, wobei bei dem Verfahren Laserstrahlung durch den Lichtleitkanal und ein Spülmediums, vorzugsweise ein Spülgas und/oder eine Spülflüssigkeit, durch den Spülkanal geleitet und durch den Absaugkanal abgesaugt wird. In einer bevorzugten Ausgestaltung des Verfahrens weist die Laserstrahlung eine Wellenlänge von 2013 ± 100 nm und/oder eine Leistung von 5 W bis 150 W auf und/oder die Laserstrahlung ist ein eine gepulste Laserstrahlung mit einer Pulslänge von 200 µs bis 1 s. In anderen Ausführungsformen der Erfindung wird ein Laser mit einer Wellenlänge von 3 µm oder ein CO₂-Laser mit einer Wellenlänge von 10,6 µm verwendet.

Mithilfe der beigefügten Zeichnungen werden nachfolgend einige Ausführungsbeispiele der vorliegenden Erfindung näher erläutert. Es zeigen
- Figuren 1 bis 4: - schematische Schnittdarstellungen durch verschiedene Ausführungsformen eines Arbeitstubus,
- Figur 5: - die schematische Darstellung eines Endoskopes und
- Figur 6: - einen vergrößerten Ausschnitt aus Figur 5.

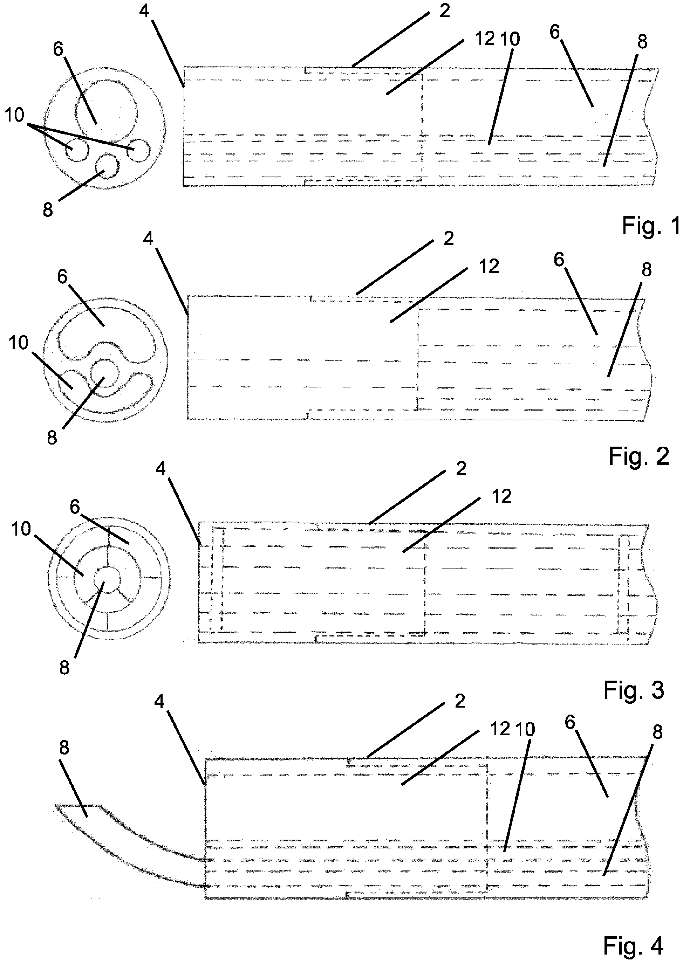

Die Figuren 1 bis 3 zeigen schematisch eine Schnittdarstellung durch einen Arbeitstubus 2, wie er in Endoskopen gemäß Ausführungsformen der vorliegenden Erfindung verwendet werden kann. Zudem zeigen die Figuren 1 bis 3 jeweils linken Teil der Figur eine frontale Ansicht auf ein vorderes Ende 4 des Arbeitstubus 2. In der Schnittdarstellung des Arbeitstubus 2 werden mit gestrichelten Linien verschiedene Kanäle dargestellt, die sich im Innern des Arbeitstubus 2 befinden.

In Figur 1 verfügt der Arbeitstubus 2 über einen Absaugkanal 6, einen Lichtleitkanal 8 sowie 2 Spülkanäle 10. Man erkennt im linken Bereich der Figur 1, dass der Absaugkanal 6 von allen Kanälen den größten Querschnitt aufweist. Die beiden Spülkanäle 10 sind rechts und links des Lichtleitkanals 8 angeordnet. Durch sie wird ein Spülgas oder eine Spülflüssigkeit in den Arbeitsraum geleitet, der sich vor dem vorderen Ende 4 des Arbeitstubus 2 befindet. Das Spülgas oder die Spülflüssigkeit tritt also aus den Spülkanäle 10 aus und wird durch den Absaugkanal 6 wieder aus dem Arbeitsraum entfernt. Durch die Anordnung der Spülkanäle 10 zum Absaugkanal 6 wird gewährleistet, dass aus dem Lichtleitkanal 8 austretendes Laserlicht, das zur Bearbeitung des Materials im Arbeitsraum verwendet wird, nicht durch das Spülgas oder die Spülflüssigkeit beeinträchtigt wird. Das vordere Ende 4 des Arbeitstubus 2 wird durch ein Abschlussstück 12 verschlossen, das ebenfalls durch eine gestrichelte Linie dargestellt ist. Der Absaugkanal 6, der Lichtleitkanal 8 und die Spülkanäle 10 verlaufen durch das Abschlussstück 12 hindurch und werden vorzugsweise durch das Abschlussstück 12 stabilisiert.

Figur 2 zeigt eine andere Ausgestaltung. Auch hier ist das vordere Ende 4 des Arbeitstubus 2 durch das Abschlussstück 12 verschlossen, durch das der Absaugkanal 6, der Lichtleitkanal 8 sowie der Spülkanal 10 hindurchgeführt ist. Man erkennt im linken Teil der Figur 2 jedoch, dass die Anordnung der verschiedenen Kanäle im Vergleich zur Darstellung aus Figur 1 unterschiedlich ist. Der Lichtleitkanal 8 ist fast zentral angeordnet und wird vom Absaugkanal 6 und vom Spülkanal 10 zumindest teilweise umgeben. Dadurch werden die Strömungseigenschaften verändert, sodass durch den Spülkanal 10 austretendes Spülgas oder Spülflüssigkeit, das durch den Absaugkanal 6 den Arbeitsraum wieder verlässt, dass durch den Lichtleitkanal 8 austretendes Licht möglichst wenig beeinträchtigt.

Figur 3 zeigt eine weitere Ausgestaltung eines durch das Abschlussstück 12 verschlossenen vorderen Endes 4 eines Arbeitstubus 2. Man erkennt im linken Teil der Figur 3, dass die verschiedenen Arten der Kanäle konzentrisch angeordnet sind. Zentral ist der Lichtleitkanal 8 positioniert. Dieser wird von einem Spülkanal 10 umgeben, der aus drei Segmenten aufgebaut ist, die den Lichtleitkanal 8 im gezeigten Ausführungsbeispiel vollständig umgeben. Die jeweils zwischen benachbarten Segmenten dargestellten Striche entsprechen lediglich Stabilisierungswänden, durch die dem Abschlussstück 12 Stabilität verliehen wird. Radial außen befinden sich vier Absaugkanal 6, die den äußeren Kanalring bilden.

Figur 4 zeigt eine Darstellung ähnlich der Ausführungsform aus Figur 1. Der Hauptunterschied zu dieser besteht jedoch darin, dass der Lichtleitkanal 8 über das vordere Ende 4 des Arbeitstubus 2 hinaus steht und dort einen gekrümmten Verlauf aufweist. So kann erreicht werden, dass aus dem Lichtleitkanal 8 austretendes Licht nicht entlang der Längsrichtung des Arbeitstubus, in den Figuren 1 bis 4 also von rechts nach links, sondern in einem anderen Winkel austritt.

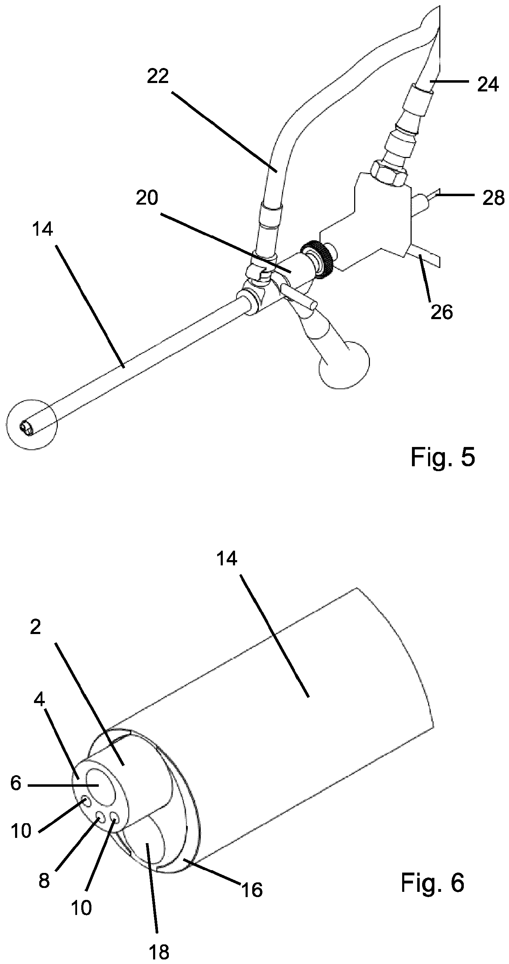

Figur 5 zeigt schematisch ein Endoskop gemäß einem Ausführungsbeispiel der vorliegenden Erfindung. Figur 6 zeigt einen vergrößerten Ausschnitt aus Figur 5, nämlich das vordere Ende des Endoskopes. Das Endoskop verfügt über ein äußeres Hüllrohr 14, in das der Arbeitstubus 2 eingeschoben ist. In Figur 6 ist das vordere Ende dargestellt. Man erkennt den Arbeitstubus 2, an dessen vorderen Ende der Lichtleitkanal 8, der Absaugkanal 6 sowie die beiden Spülkanäle 10 zu erkennen sind. Zusätzlich sind in dem äußeren Hüllrohr 14 ein Beleuchtungskanal 16 sowie ein Beobachtungskanal 18 angeordnet. Durch den Beleuchtungskanal 16 wird Beleuchtungslicht in den vor dem vorderen Ende 4 liegenden Arbeitsraum geleitet, sodass der Operateur bei der Verwendung des Endoskopes den Bearbeitungsraum beobachten kann. Das Beleuchtungslicht beleuchtet den Arbeitsraum und reflektiertes Beleuchtungslicht tritt durch den Beobachtungskanal 18 wieder in das äußere Hüllrohr 14 ein.

In Figur 5 ist dargestellt, dass sich am hinteren Ende des äußeren Hüllrohr 14 ein Koppelstück 20 befindet, in dem die verschiedenen Kanäle, die sich im äußeren Hüllrohr 14 und insbesondere im Arbeitstubus 2 befinden mit verschiedenen Versorgungsleitungen verbunden werden. Dazu verfügt das Endoskop über eine Lichtleitung 22, die an ihrem nicht gezeigten Ende vorzugsweise mit einer Lichtquelle verbunden ist. Diese Lichtquelle sendet Beleuchtungslicht aus, das durch die Lichtleitung 22 zum Koppelstück 20 und von dort in den Beleuchtungskanal 16 geleitet wird. Ein Absauganschluss 24 ist vorzugsweise mit einer Unterdruckquelle, beispielsweise einer Pumpe, verbunden. Diese erzeugt einen Unterdruck und damit eine Sogwirkung, die über das Koppelstück 20 auf den Absaugkanal 6 übertragen wird. Material, das durch den Absaugkanal 6 abgesaugt wird, wird über das Koppelstück 20 dem Absauganschluss 24 zugeführt. Spülgas oder Spülflüssigkeit wird über einen Spülanschluss 26 dem Koppelstück 20 und darüber dem Spülkanal 10 zugeführt. Das für die Laserbearbeitung notwendige Laserlicht, das über den Lichtleitkanal 8 dem Arbeitsraum zugeführt wird, gelangt über einen Laseranschluss 28 in das Koppelstück 20 und von dort in den Lichtleitkanal 8.

### Bezugszeichenliste

2 Arbeitstubus
4 vorderes Ende
6 Absaugkanal
8 Lichtleitkanal
10 Spülkanal
12 Abschlussstück
14 äußeres Hüllrohr
16 Beleuchtungskanal
18 Beobachtungskanal
20 Koppelstück
22 Lichtleitung
24 Absauganschluss
26 Spülanschluss
28 Laseranschluss

## Patentansprüche

1. Endoskop mit einem äußeren Hüllrohr (14), in dem
ein Arbeitskanal mit einer vorderen Öffnung und einer hinteren Öffnung und
einer Längsrichtung, die von der hinteren Öffnung zu der vorderen Öffnung verläuft, verläuft,
**dadurch gekennzeichnet, dass**
das Endoskop einen Arbeitstubus (2) mit einem vorderen Ende (4) und einem hinteren Ende aufweist, der
in die hintere Öffnung des Arbeitskanals einschiebbar ist und
wenigstens einen Lichtleitkanal (8) zum Leiten von Laserstrahlung,
wenigstens einen Spülkanal (10) zum Leiten eines Spülgases und/oder einer Spülflüssigkeit und
wenigstens einen Absaugkanal (6) aufweist.

2. Endoskop nach Anspruch 1, **dadurch gekennzeichnet, dass** der Lichtleitkanal (8) als Lichtleitfaser, insbesondere Glasfaser, und/oder als Hohlleiter und/oder als Freistrahl ausgebildet sind.

3. Endoskop nach Anspruch 1 oder 2, **dadurch gekennzeichnet, dass** der Lichtleitkanal (8) zum vorderen Ende (4) des Arbeitstubus (2) hin durch ein optisches Bauteil, insbesondere eine Linse oder ein Prisma, verschlossen ist.

4. Endoskop nach Anspruch 3, **dadurch gekennzeichnet, dass** das optische Bauteil so ausgebildet ist, dass Licht, das durch den Lichtleitkanal (8) geleitet wird und durch das optische Bauteil hindurchtritt, entlang einer Austrittsrichtung, die einen Winkel von wenigstens 10°, bevorzugt wenigstens 30° mit der Längsrichtung des Arbeitskanals einschließt, aus dem optischen Bauteil austritt.

5. Endoskop nach einem der vorstehenden Ansprüche, **dadurch gekennzeichnet, dass** der Lichtleitkanal (8) in einem Abschnitt des Arbeitstubus (2) nicht parallel zum Arbeitstubus (2) verläuft, wobei der Abschnitt vorzugsweise unmittelbar vor dem vorderen Ende (4) liegt.

6. Endoskop nach Anspruch 5, **dadurch gekennzeichnet, dass** sich in dem Abschnitt eine Form einer Querschnittsfläche des Spülkanals (10) und/oder des Absaugkanals (6) ändert, wobei vorzugsweise der Flächeninhalt der Querschnittsfläche konstant ist.

7. Endoskop nach einem der vorstehenden Ansprüche, **dadurch gekennzeichnet, dass** in dem äußeren Hüllrohr (14), vorzugsweise in dem Arbeitstubus (2) eine Beobachtungseinrichtung vorhanden ist, die eingerichtet ist, Licht zu detektieren, das aus einem vorbestimmten Raumwinkelbereich relativ zur Längsrichtung des Arbeitskanals in die Beobachtungseinrichtung einfällt.

8. Endoskop nach Anspruch 7, **dadurch gekennzeichnet, dass** die Austrittsrichtung in dem vorbestimmten Raumwinkelbereich liegt, wobei vorzugsweise der vorbestimmte Raumwinkelbereich symmetrisch um die Austrittsrichtung ist.

9. Endoskop nach Anspruch 7 oder 8, **dadurch gekennzeichnet, dass** die Beobachtungseinrichtung einen Lichtdetektor, beispielsweise einen CCD-Chip aufweist.

10. Endoskop nach einem der vorstehenden Ansprüche, **dadurch gekennzeichnet, dass** der Arbeitskanal und/oder der Spülkanal (10) und/oder der Absaugkanal (6) als Rohr, vorzugsweise als metallisches Rohr ausgebildet sind.

11. Endoskop nach einem der vorstehenden Ansprüche, **dadurch gekennzeichnet, dass** der Arbeitstubus (2) mehrere, vorzugsweise 2, 3 oder 4, Spülkanäle (10) aufweist.

12. Endoskop nach einem der vorstehenden Ansprüche, **dadurch gekennzeichnet, dass** das vordere Ende (4) des Arbeitstubus (2) von einem Abschlussstück (12) verschlossen ist, durch das alle Kanäle hindurchgeführt sind.

13. Endoskop nach Anspruch 12, **dadurch gekennzeichnet, dass** das Abschlussstück in Längsrichtung des Arbeitskanals über das optische Bauteil am vorderen Ende des Lichtleitkanals (8) hinausragt.

14. Endoskop nach einem der vorstehenden Ansprüche, **dadurch gekennzeichnet dass** der Arbeitstubus (2) eine Arretierungseinrichtung aufweist, mit der er in mehreren vorbestimmten Orientierungen, vorzugsweise in einer einzigen Orientierung, in dem Arbeitskanal arretierbar ist.

15. Arbeitstubus (2) für ein Endoskop nach einem der vorstehenden Ansprüche.

16. Verfahren zum Betreiben eines Endoskops nach einem der vorstehenden Ansprüche, wobei bei dem Verfahren Laserstrahlung durch den Lichtleitkanal (8) und ein Spülgas und/oder eine Spülflüssigkeit durch den Spülkanal (10) geleitet und durch den Absaugkanal ()6 abgesaugt wird.

17. Verfahren nach Anspruch 16, **dadurch gekennzeichnet, dass** die Laserstrahlung eine Wellenlänge von 2013 ± 100 nm und/oder eine Leistung von 5W bis 150W aufweist und/oder eine gepulste Laserstrahlung mit einer Pulslänge von 200 µs bis 1 s ist.
